# EUROPEAN PATENT SPECIFICATION

(11) **EP 3 784 728 B1**
(45) Date of publication and mention of the grant of the patent: **06.11.2024**
(21) Application number: 19732666.3
(22) Date of filing: 25.06.2019
(51) Int. Cl.: C08K 5/12, C08K 5/3475, C08L 23/06, C08L 31/06

(54) **ISOTOPICALLY LABELLED MATERIALS FOR DEGRADATION DETECTION**
ISOTOPENMARKIERTE MATERIALIEN ZUR ERKENNUNG DES ABBAUS
MATÉRIAUX MARQUÉS ISOTOPIQUEMENT POUR DÉTECTION DE DÉGRADATION

(30) Priority: 25.06.2018 EP 18382468
(43) Date of publication of application: 03.03.2021
(73) Proprietor: INTA, Instituto Nacional de Técnica Aeroespacial, 28850 Torrejon de Ardoz (ES); Instituto Tecnológico del Plástico (Aimplas), 46980 Paterna (ES)
(72) Inventor: MORA NOGUÉS, Julio, 28050 Madrid (ES); BENEDITO BORRÁS, Adolfo, 46980 PATERNA (ES); GARCÍA SANCHO, Amador, 46019 VALENCIA (ES); ALONSO RUIZ, Rafael, 46022 VALENCIA (ES)
(74) Representative: TRBL Intellectual Property
(86) International application number: PCT/EP2019/066777
(87) International publication number: WO 2020/002297

(56) References cited:
- WO-A1-2014/124496
- CN-A- 104 140 523
- CN-A- 106 188 132
- US-A1- 2007 282 145
- US-A1- 2010 062 251
- US-A1- 2010 063 208
- US-A1- 2015 377 841
- XUDONG YAO ET AL: "Dissection of proteolytic O-18 labeling: Endoprotease-catalyzed O-16 to O-18 exchange of truncated peptide substrates", JOURNAL OF PROTEOME RESEARCH, AMERICAN CHEMICAL SOCIETY, vol. 2, no. 2, 1 January 2003 (2003-01-01), pages 147 - 152, XP002658376, ISSN: 1535-3893, [retrieved on 20021211], DOI: 10.1021/PR025572S
- SCHMIDT O ET AL: "A SIMPLE UREA LEAF-FEEDING METHOD FOR THE PRODUCTION OF 13C AND 15N LABELLED PLANT MATERIAL", PLANT AND SOIL, KLUWER ACADEMIC PUBLISHERS, DORDRECHT, NL, vol. 229, no. 2, 1 February 2001 (2001-02-01), pages 197 - 202, XP009013544, DOI: 10.1023/A:1004815404197

## Description

### FIELD OF INVENTION

The present invention belongs to the field of material contamination or degradation detection, particularly to the use of isotopically labelled materials for the detection of material contamination in industrial processes or in space research.

### BACKGROUND OF THE INVENTION

The space exploration and the search of life in other celestial bodies has suffered an exponential increase in the last 60 years. Technological advances have made it possible to set objectives and achieve goals that were unthinkable before 1957, the date on which the first successful satellite, Sputnik 1, was launched. This event launched the space race, and with it, a large number of missions that have explored planets, satellites and comets from our galaxy.

The beginning of these contacts brought with it concern about the possible contamination produced in the visited bodies (Forward contamination), and that received in the return missions (Backward contamination). To study how to minimize the effect of these processes and issue recommendations the COSPAR (Committee on space research) was formed in 1964. Its resolutions were ratified by the United Nations in the "External Space Treaty" of 1967. Space agencies have adopted these recommendations in their procedures and the Planetary Protection protocols are strictly followed in all space missions. The cleaning procedures and the control systems for biological, molecular and particle contamination are very rigorous and are present in all phases of the mission: design, manufacture, assembly, integration, testing, storage, transportation, preparation for launch, launch and orbit.

Especially sensitive to cross contamination are the scientific missions *in situ* whose objective is the search for life precursors. The analytical equipment shipped aboard the rovers and probes are increasingly sensitive and the detection range is becoming smaller (ppb). A natural pollution, or accidental, produced by the material transported from the Earth in a mission can produce a false positive in the search for life precursors, where biological traces as simple as C-H, C-O or C-N links are looked for.

Advances in polymer technology have allowed to improve the mechanical and thermal properties, which together with its lightness, has made polymers very interesting candidates for spatial use as structural materials. As functional materials its use is even more widespread: wiring, adhesives, plastic connectors, lubricants or gaskets that are present in any mission.

However, the simple signals of the mentioned links could be detected in a large number of the polymers, which are the fundamental basis of their composition, and in case of contamination, produce a false positive in the analysis of the samples.

US 2010/063208 A1 and US 2010/062251 A1 relate to taggant fibers made of polymers selected from polyolefins for enhanced protection and security when said fibers are used in documents such as land titles, currency, passports and other documents of value. US 2015/377841 A1, CN 104140523 A, WO 2014/124496 A1, CN 106188132 A, US 2007/282145 A1, disclose different isotopically labelled polymer materials. Whether these materials are suitable for the detection of material contamination, degradation, or wear under industrial or space environments is unknown.

### SUMMARY OF THE INVENTION

The present invention provides a material which allows the detection of any contamination or degradation or wear of said material in a simple and very reliable way. The inventors of the present invention have found that an isotopically marked functional material can be traced and, moreover, that its different components can be traced so as to identify if there has been any contamination or degradation of the material in general or of any of its components in particular.

In a first aspect, the present invention relates to the use of a material comprising a synthetic functional polymer and optionally at least one functional additive in the detection of material contamination or degradation or wear, wherein said material is marked with at least one isotope, wherein the isotope or isotopes are present in a functional component of the material and wherein the at least one isotope is selected from ²H, ¹³C, ¹⁵N, ¹⁷O,¹⁸O, ²⁹Si, ³⁰Si, ³³S, ³⁴S, ³⁶S, ³⁷Cl, wherein said material is a space material or an industrial material, preferably wherein the industrial material is a material to be used in a clean room. Said functional component or components of the material where the isotope or isotopes are present is not used in the material for marking said material but has another function in said material other than marking the material, such as a structural function, or a function such as that of a plasticizer, a flame retardant, a filler, an antioxidant, a metal scavenger, a UV protector, a photostabilizer, a heat stabilizer, an impact modifier, etc. Thus, the marked functional component is not present in the material only for the purpose of labelling or tagging the material.

### DESCRIPTION OF THE INVENTION

In a preferred embodiment of the first aspect, the present invention relates to the use of a material comprising at least one synthetic functional polymer and optionally at least one functional additive in the detection of material contamination or degradation or wear, wherein said material is marked with at least one isotope, wherein the isotope or isotopes are present in a functional component of the material, and wherein the at least one isotope is selected from ²H, ¹³C, ¹⁵N, ¹⁷O, ¹⁸O, ²⁹Si, ³⁰Si, ³³S, ³⁴S, ³⁶S, ³⁷Cl, wherein said material is a space material or an industrial material, preferably wherein the industrial material is a material to be used in a clean room. These isotopes form covalent bonds in organic compounds.

In a preferred embodiment of the first aspect, the material comprises more than one component and the same isotope is used for marking different components.

**Table 1. Isotopes.**

| Isotopes | | | | | | |
|---|---|---|---|---|---|---|
| 1^{H} | ³⁶Ar | ⁶⁸Zn | ⁹⁷Mo | ¹²³Sn | ¹⁵⁰Sm | ¹⁷⁸Hf |
| ²H | ³⁸Ar | ⁷⁰Zn | ⁹⁸Mo | ¹²⁰Te | ¹⁵²Sm | ¹⁷⁹Hf |
| ³He | ⁴⁰Ar | ⁶⁹Ga | ⁹⁶Ru | ¹²²Te | ¹⁵⁴Sm | ¹⁸⁰Hf |
| ⁴He | ³⁹K | ⁷¹Ga | ⁹⁸Ru | ¹²³Te | ¹⁵³Eu | ¹⁸¹Ta |
| ⁶Li | ⁴¹K | ⁷⁰Ge | ⁹⁹Ru | ¹²⁴Te | ¹⁵⁴Gd | ¹⁸²W |
| 7^{L}i | ⁴⁰Ca | ⁷²Ge | ¹⁰⁰Ru | ¹²⁵Te | ¹⁵⁵Gd | ¹⁸³W |
| ⁹Be | ⁴²Ca | ⁷³Ge | ¹⁰¹Ru | ¹²⁶Te | ¹⁵⁶Gd | ¹⁸⁴W |
| ¹⁰B | ⁴³Ca | ⁷⁴Ge | ¹⁰²Ru | ¹²⁷I | ¹⁵⁷Gd | ¹⁸⁶W |
| ¹¹B | ⁴⁴Ca | ⁷⁵As | ¹⁰⁴Ru | ¹²⁴Xe | ¹⁵⁸Gd | ¹⁸⁵Re |
| ¹²C | ⁴⁶Ca | ⁷⁴Se | ¹⁰³Rh | ¹²⁶Xe | ¹⁶⁰Gd | ¹⁸⁴Os |
| ¹³C | ⁴⁵Sc | ⁷⁶Se | ¹⁰²Pd | ¹²⁸Xe | ¹⁵⁹Tb | ¹⁸⁷Os |
| ¹⁴C | ⁴⁶Ti | ⁷⁷Se | ¹⁰⁴Pd | ¹²⁹Xe | ¹⁵⁶Dy | ¹⁸⁸Os |
| ¹⁴N | ⁴⁷Ti | ⁷⁸Se | ¹⁰⁵Pd | ¹³⁰Xe | ¹⁵⁸Dy | ¹⁸⁹Os |
| ¹⁵N | ⁴⁸Ti | ⁸⁰Se | ¹⁰⁶Pd | ¹³¹Xe | ¹⁶⁰Dy | ¹⁹⁰Os |
| ¹⁶O | ⁴⁹Ti | ⁷⁹Br | ¹⁰⁸Pd | ¹³²Xe | ¹⁶¹Dy | ¹⁹²Os |
| ¹⁷O | ⁵⁰Ti | ⁸¹Br | ¹¹⁰Pd | ¹³⁴Xe | ¹⁶²Dy | ¹⁹¹Ir |
| ¹⁸O | ⁵¹V | ⁸⁰Kr | ¹⁰⁷Ag | ¹³³Cs | ¹⁶³Dy | ¹⁹³Ir |
| ¹⁹F | ⁵⁰Cr | ⁸²Kr | ¹⁰⁹Ag | ¹³²Ba | ¹⁶⁴Dy | ¹⁹²Pt |
| ²⁰Ne | ⁵²Cr | ⁸³Kr | ¹⁰⁶Cd | ¹³⁴Ba | ¹⁶⁵Ho | ¹⁹⁴Pt |
| ²¹Ne | ⁵³Cr | ⁸⁴Kr | ¹⁰⁸Cd | ¹³⁵Ba | ¹⁶²Er | ¹⁹⁵Pt |
| ²²Ne | ⁵⁴Cr | ⁸⁶Kr | ¹¹⁰Cd | ¹³⁶Ba | ¹⁶⁴Er | ¹⁹⁶Pt |
| ²³Na | ⁵⁵Mn | ⁸⁵Rb | ¹¹¹Cd | ¹³⁷Ba | ¹⁶⁶Er | ¹⁹⁸Pt |
| ²⁴Mg | ⁵⁴Fe | ⁸⁴Sr | ¹¹²Cd | ¹³⁸Ba | ¹⁶⁷Er | ¹⁹⁷Au |
| ²⁵Mg | ⁵⁶Fe | ⁸⁶Sr | ¹¹⁴Cd | ¹³⁹La | ¹⁶⁸Er | ¹⁹⁶Hg |
| ²⁶Mg | ⁵⁷Fe | ⁸⁷Sr | ¹¹³In | ¹³⁶Ce | ¹⁷⁰Er | ¹⁹⁸Hg |
| ²⁷Al | ⁵⁸Fe | ⁸⁸Sr | ¹¹²Sn | ¹³⁸Ce | ¹⁶⁹Tm | ¹⁹⁹Hg |
| ²⁸Si | ⁵⁹Co | ⁸⁹Y | ¹¹⁴Sn | ¹⁴⁰Ce | ¹⁶⁸Yb | ²⁰⁰Hg |
| ²⁹Si | ⁵⁸Ni | ⁹⁰Zr | ¹¹⁵Sn | ¹⁴²Ce | ¹⁷⁰Yb | ²⁰¹Hg |
| ³⁰Si | ⁶⁰Ni | ⁹¹Zr | ¹¹⁶Sn | ¹⁴¹Pr | ¹⁷¹Yb | ²⁰²Hg |
| ³¹P | ⁶¹Ni | ⁹²Zr | ¹¹⁷Sn | ¹⁴²Nd | ¹⁷²Yb | ²⁰⁴Hg |
| ³²S | ⁶²Ni | ⁹⁴Zr | ¹¹⁸Sn | ¹⁴³Nd | ¹⁷³Yb | ²⁰³Tl |
| ³³S | ⁶⁴Ni | ⁹³Nb | ¹¹⁹Sn | ¹⁴⁵Nd | ¹⁷⁴Yb | ²⁰⁵Tl |
| ³⁴S | ⁶³Cu | ⁹²Mo | ¹²⁰Sn | ¹⁴⁶Nd | ¹⁷⁶Yb | ²⁰⁴Pb |
| ³⁶S | ⁶⁵Cu | ⁹⁴Mo | ¹²²Sn | ¹⁴⁸Nd | ¹⁷⁵Lu | ²⁰⁶Pb |
| ³⁵Cl | ⁶⁴Zn | ⁹⁵Mo | ¹²⁴Sn | ¹⁴⁴Sm | ¹⁷⁶Hf | ²⁰⁷Pb |
| ³⁶Cl | ⁶⁶Zn | ⁹⁶Mo | ¹²¹Sb | ¹⁴⁹Sm | ¹⁷⁷Hf | ²⁰⁸Pb |
| ³⁷Cl | ⁶⁷Zn | | | | | |

In another preferred embodiment of the first aspect, the material comprises more than one component and wherein a different isotope is used for marking different components.

In a preferred embodiment of the first aspect, the isotope is introduced in a specific position in a monomer of the synthetic polymer.

The term "functional" as used herein means that the synthetic polymer or the additive's purpose or function is not exclusively marking the material, that is, the synthetic polymer or additive has a function other than marking the material. For example, the function of the synthetic polymer may be structural. For example, the function of the additive may be a plasticizer, a flame retardant, a filler, an antioxidant, a metal scavenger, a UV protector, a photostabilizer, a heat stabilizer, or an impact modifier. The term "functional" as used herein should not be understood as "functional group" but as explained above.

The expression "present in a functional component of the material" also means that the component of the material which is isotopically marked has a function other than marking the material. For example, when the component that is isotopically marked is the synthetic polymer, this polymer may be a structural component, useful for its mechanical properties, or a functional component, useful for its chemical, magnetic, electronic properties, etc., and this polymer will be useful for other reasons than for being marked.

The term "component" as used herein means any constituting part of a larger whole, any constituent. In the present description, the term "component" refers to the material and, therefore, refers to any constituting part of the material.

The term "marked", or "marking" as used herein means that the material in general and the marked component in particular, comprise a different isotopic ratio than the isotopic ratio present in the medium or environment where the material is used. For example, for space material to be used in Mars, the isotopic environment in the material will be different than the isotopic environment in Mars. For space material to be used in the Moon, the isotopic environment in the material will be different than the isotopic environment in the Moon. For a prosthetic material to be used in the human body, the isotopic environment in the material will be different than the isotopic environment in the human body. The skilled person is fully aware of how to prepare the materials of the present invention, once the particular isotopic environment for the material has been chosen (see for example Nikonowicz, E. P. et al. 1992 Nucleic acids research, 20 (17), 4507-4513; Schmidt, O., and Scrimgeour, C. M. (2001). Plant and Soil, 229(2), 197-202; Liu, L., and Fan, S. (2001) Journal of the American Chemical Society, 123(46), 11502-11503; Mulder, F. M. et al. 1998 Journal of the American Chemical Society, 120(49), 12891-12894; US 6,541,671; Park, S. et al (2012). Nature communications, 3, 638; Connolly, B. A., and Eckstein, F. (1984). Biochemistry, 23(23), 5523-5527; Crosby, S. R., et al. (2002). Organic letters, 4(20), 3407-3410; Yao, X. et al. (2003) Journal of proteome research, 2(2), 147-152).

The terms "labelled" and "marked" are used interchangeably in the present description.

The expression "isotopic environment" as used herein refers to the percentage of each isotope of each chemical element in a certain physical environment, i.e. in a certain planet, satellite, etc. The expression "different isotopic environment" as used herein means that upon detecting the percent of a certain isotope of a certain chemical element in the material and in a particular natural environment, different percentages will be obtained. For example, for a material marked with ²H (deuterium) to be used in Mars, its minimum mark will be 5 times the abundance of ²H in Mars, which is 0.3895 % of the Hydrogen atoms in the marked component of the material will be ²H.

For example, the plasticizer dioctyl phthalate (DOP) can be added in a 0.1 weight % to the composition of a material comprising a synthetic polymer. If DOP is marked at the 50 % of a set atomic position, this means that this component of the material is marked and if it degasifies, the degraded component will be detected because of the different signals generated by this 50% of marked positions.

The present invention allows to have different marking in each component which allows to identify the component which is suffering degradation.

A material can be 100% traceable if all of its components are marked and each one is marked using a specific marking, which can be associated to a specific component or material upon detection.

The expression "industrial material" as used herein refers to any material suitable for industrial applications. Materials suitable for industrial applications must be validated according to the characteristics of the specific field of use. Two examples of industrial material are:
- critical components of a loop system where the degradation of these components needs to be evaluated for maintenance or monitoring purposes.
- controlled environments (i.e.: clean rooms) where the contamination needs to be monitored and the contaminants need to be identified.

The expression "space material" as used herein refers to any material suitable for a space mission. Materials suitable for space missions must be validated according to the requirements of each mission in terms of space environment effects, such as vacuum, heat, thermal cycling, radiation, debris, etc. and in terms of induced space environment effects, such as contamination, secondary radiations and spacecraft charging. These space environment effects are defined by the external physical world for each mission: atmosphere, meteoroids, energetic particle radiation, etc. The induced space environment is that set of environmental conditions created or modified by the presence or operation of the item and its mission. The space environment also contains elements which are induced by the execution of other space activities (e.g. debris and contamination).

In a preferred embodiment of the first aspect, at least 0.3 % of the atoms of the chemical element of the isotope are marked, in respect of the total number of atoms of that chemical element in the marked component of the material. Preferably, at least 0.5 % of the atoms of the chemical element of the isotope are marked, in respect of the total number of atoms of that chemical element in the marked component of the material. More preferably, at least 1 % of the atoms of the chemical element of the isotope are marked, in respect of the total number of atoms of that chemical element in the marked component of the material. In a more preferred embodiment, at least 2 % of the atoms of the chemical element of the isotope are marked, in respect of the total number of atoms of that chemical element in the marked component of the material. In an even more preferred embodiment, at least 5 % of the atoms of the chemical element of the isotope are marked, in respect of the total number of atoms of that chemical element in the marked component of the material. In another embodiment, at least 30 % of the atoms of the chemical element of the isotope are marked, in respect of the total number of atoms of that chemical element in the marked component of the material. The minimum marking of the material will depend on the technique intended to be used for detection and its sensitivity.

In a preferred embodiment of the first aspect, the isotopic mark is detected by FTIR, Raman, GC/MS, RMN-H, RMN-C, UV-visible spectroscopy. The isotopic mark is detected by any analytical technique that can detect the differences between the natural isotopic environment and the induced isotopic environment in the material. Preferably, the isotopic mark is detected by FTIR, Raman, GC/MS, RMN-H, RMN-C and/or UV-visible spectroscopy. More preferably, the isotopic mark is detected by Raman or GC/MS.

The materials of the present invention are characterized physico-chemically analysing their TGA, DSC, degree of crystallinity, glass transition temperature, gel permeation chromatography (GPC), FTIR, Raman and H-NMR. The degradation/contamination/wear of the materials of the present invention can be detected by means of the same analytical techniques used in the rover of the Exomars 2020 mission: Raman, GC/MS, etc. For example, the analytical techniques used in Martian rovers to search organic life signatures are gas chromatography with mass spectroscopy (GC/MS), laser desorption with mass spectroscopy (LD/MS) and Raman spectroscopy.

For those materials to be used in space, said materials will undergo the relevant spatial validation tests, required for all materials that participate in space missions, and which are determined by the type of mission, the function of the component, and its exposure to environmental agents.

For the materials described in the present invention, the rules of the ESA (European Space Agency) have been followed, and the validation tests have been those determined by the following standards:
- ECSS-E-ST-10-03C, "Space Engineering-Testing".
- ECSS-Q-ST-70C, "Space product assurance- Materials, mechanical parts and processes".

In a preferred embodiment of the first aspect, the synthetic polymer is an addition polymer or a condensation polymer. Preferably, the synthetic polymer is a polyolefin, a polyester, a polyurethane, a polyimide, a polyacrylate, a polysiloxane, a polyepoxide, a fluorinated polymer or a combination thereof. more preferably, the synthetic polymer is polyethylene (PE), polyethylene terephthalate (PET), polyamide (PA), ethylene tetrafluoroethylene (ETFE), polytetrafluoroethylene (PTFE), perfluoroalkoxy alkane (PFA), polyetheretherketone (PEEK), polyethersulphone (PES), polysulfone, polyetherimide (PEI) or a copolymer o terpolymer thereof.

Examples of synthetic bioabsorbable polymers that may be used for prosthetic materials are polyglycolide, or polyglycolic acid (PGA), polylactide, or polylactic acid (PLA), poly ε-caprolactone, polydioxanone, polylactide-co-glycolide, e.g., block or random copolymers of PGA and PLA, and other commercial bioabsorbable medical polymers. Preferred is spongy collagen or cellulose.

In a preferred embodiment of the first aspect, the material is a plastic, an adhesive, a coating, a varnish, a tape, a film, a paint, an ink, a lubricant, a potting, a sealant, a foam, a rubber, a wire or a cable.

In a preferred embodiment of the first aspect, the material is used as a calibrator for a Raman spectrometer.

In a preferred embodiment of the first aspect, the material is comprised in a composite material. Preferred composite materials comprise at least one of carbon fibre, polyethylene, polypropylene, nylon or kevlar. Preferably, the composite material comprises a binder and reinforcement fibres and/or particles. Said binder and/or reinforcement fibres and/or particles could be also polymeric.

In a preferred embodiment of the first aspect of the present invention, the material comprises different components and all the marked components are marked with the same isotope.

### EXAMPLES

In order to provide a better understanding of the invention, the following is a detailed explanation of some of the preferred embodiments of the invention, which is provided to give an illustrative example of the invention but which, by no means, should be considered to limit the same.

### Example 1

As an example of isotopically labelled structural or functional material for the application of space contamination detection, PET polymers (Polyethylene terephthalate) have been synthesized and have the same technical characteristics as the PET used as the calibrator of the Raman spectrometer that will go aboard the Exomars. These polymers have been synthesized starting from:
- Precursor monomer 1: Ethylene glycol and its deuterated namesake (ethylene-d₄ glycol)
- Precursor monomer 2: terephthaloyl chloride and its deuterated namesake (terephthaloyl-d₄ chloride)
- Solvents: chloroform/H₂O
- Additives: Surfactant Hexadecyltrimethylammonium bromide (CTAB).
- NaOH is added as a base.

The synthesis has been carried out by additive polymerization (polycondensation in interface), in a two-phase system composed of an organic and an inorganic phase, with the following conditions:
- Temperature: 50 °C
- Stirring: ≈ 500 rpm.
- Pressure, vacuum: atmospheric pressure.
- Catalyst: Not used.
- Time: ≈ 20 hours.
- Type of atmosphere: air.
- molar ratio of the monomers: 1:1.

The monomers were added in a staggered manner in two independent phases. The interfacial polymerization proceeded then in the following way:
- First, the deionized H₂O was stirred together with the suitable amount of NaOH. When dissolved, ethylene glycol and surfactant (CTAB) were added. When a homogeneous solution was achieved (between 2-10 minutes), the next phase, consisting in the corresponding amount of terephthaloyl chloride dissolved in chloroform, was added.
- The two phases were mixed and maintained with vigorous stirring for approximately 20 hours at 50 °C.

The ratio of isotopically labelled polymer was graduated by employing different mixtures of the monomers and their deuterated analogues. For the example, 5 different compositions were made:
- 0% labelling: 1X mol of terephthaloyl chloride + 1Y mol of ethylene glycol.
- 10% labelling: 0.9X mol of terephthaloyl chloride + 0.1X mol of terephthaloyl-d₄ chloride + 0.9Y mol of ethylene glycol + 0.1Y mol of ethylene-d₄ glycol.
- 25% labelling: 0.75X mol of terephthaloyl chloride + 0.25X mol of terephthaloyl-d₄ chloride + 0.75Y mol of ethylene glycol + 0.25Y mol of ethylene-d₄ glycol.
- 50 % labelling: 0.5 X mol of terephthaloyl chloride + 0.5 X mol of terephthaloyl-d₄ chloride + 0.5 Y mol of ethylene glycol + 0.5 Y mol of ethylene-d₄ glycol.
- 100% labelling: X mol of terephthaloyl-d₄ chloride + Y mol of ethylene-d₄ glycol.

### Purification:

The purification of the resulting material was carried out in the following manner:
- Once the reaction time has elapsed, the resulting product was washed three times filtered and collected. The purified product was then introduced in a stove until it was completely dried.

After this purification, it was necessary to carry out a bakeout to release the non-crosslinked monomers, and the residues of additives and solvent, typical in materials for space use.

### Example 2: Synthesis of deuterium marked polyethylene terephthalate.

Over a solution of 0.001 to 17 kg of NaOH (0.30 mol/L) in water, 0.0035 to 500 mol of a mixture of ethyleneglycol and ethylene-d₄ glycol (ratio from 0 to 100 %; total concentration 0.41 mol/L) was added under stirring at a moderate speed. Subsequently, 0.01 mol-% of phase transfer catalyst (for example, tetrabutylammonium bromide) dissolved in 0.001 to 10 liters of water were added. A mixture of terephthaloyl chloride and terephthaloyl-d₄ chloride (ratio from 0 to 100 %; molar ratio diol/diacid chloride 1:1) was dissolved in chloroform (ratio water/chloroform 70:30). The organic phase was then added over the aqueous layer under vigorous stirring and mixing continued for 5 to 60 minutes. Acetone was added to the reaction vessel and the polymer was filtered off and washed with acetone to remove unreacted monomers. The material was subsequently washed three times with water and then filtered off. The final product was dried to constant weight in a vacuum oven at 40 °C.

### Example 3: Synthesis of deuterium marked polyethylene.

Ethylene and ethylene-d₄ were introduced at different ratios and at a moderate flow to a stirred solution containing a 1 to 1 mixture of TiCl₄ and AlEt₃ in hexanes under N₂ atmosphere. When the reaction mixture became thick, the mixture was hydrolyzed by addition of several amounts of ethanol. The resulting material was subsequently washed several times with ethanol, filtered and dried.

### Example 4: identification of marked PET

The PET polymer was marked using deuterium in the 100% of the hydrogen atomic positions of both precursor monomers (ethylenglycol-d4, and terephthaloyl chloride-d4).

In order to detect/identify the marked PET, different techniques were used:

### Raman spectroscopy

Raman spectroscopy is a non-destructive technique that does not need the previous preparation of the sample.

For this study the Raman spectrometer used was a RAMAN Horiba XPlora with Laser: 532 nm (Green) and Confocal microscope 10x.

We found that the isotopic substitution of deuterium (²H) instead of protium (¹H) in the 100% of hydrogen positions (aliphatic and aromatic) in PET caused little differences in many of the detected signals, but in those in which the hydrogen interaction was higher, the shift of the signals was more notorious and easy to differentiate in the marked sample. Some examples of the most representative were the following:

**Table 2. Summary of main differences detected by marking the PET polymer.**

| **Interpretation of band** | **Protium (cm⁻¹)** | **Deuterium (cm⁻¹)** |
|---|---|---|
| C-H stretching aromatic | 3089 | 2302, 2285 |
| C-H stretching aliphatic | 2973, 2944, 2928, 2854 | 2218, 2150, 2107 |
| Ring C₁-C₄ stretching | 1615, 1310, 1192, 800, 701 | 1575, 1024, 847, 756, 689, 622 |
| CH₂ bending and CCH bending in the ethylene glycol segments | 1462, 1418 | 1125, 1000 |
| O-CH₂ and C-C stretch of the Trans ethylene glycol unit | 1002 | 893 |

Only the deuterated signals (Table 2) appeared in the spectrum, clearly differentiated from the analogous protium examples that did not appear in that case. The ratio of the intensity of equivalent signals must be proportional to the ratio of the marked:non-marked positions.

In the case of 50% of marked positions in both precursor monomers, the protium and deuterium signals will appear in the spectrum with same intensity, but keeping the shift that allows to differentiate.

### Gas chromatography and mass spectrometry (GC/MS)

50 mg of the powdered 100% marked PET was dissolved in 1 ml of acetone for HPLC (≥99.9%).

The GC/MS system used was a Varian Saturn 2200. The parameters of the method were:
- Chromatographic parameters: Column WCOT Fused Silica Rapid-MS, 10 m x 0,53 mm, od: 0,25 µm; 40°C (8 min), and then increase to 250 °C at 10 °C/min. to keep at 250 °C during 37 min. Injector 1177 a 280 °C and 1:5 split (1 µl injected). Carrier gas: Helium at 1.0 ml/min.
- MS parameters: ion trap, with ionization mode: electronic impact (EI), scanning in the range 30-650 M/z, 2 scan/second.

1 µl of the PET/acetone solution was injected directly in the 1177 injector of the GC, using a 5 µl Hamilton syringe.

As it happened in the Raman study, only the mass of the deuterated fragments (table 3) appeared in the chromatogram.

The +1(M/z) caused by every deuterium introduced instead of a protium has an accumulative effect, and in the PET case, the 8 marked position generates a +8 (M/z) for the molecular ion, and at least +4(M/z) in the most of identification fragments:

**Table 3. Summary of main differences in the mass (M/z) of fragments detected by marking the PET polymer.**

| **Fragment** | **Protium (cm⁻¹)** | **Deuterium (cm⁻¹)** |
|---|---|---|
| [CH₂-OH]⁺ | **31**/33 | **33**/35 |
| [CH₂-OH]₂⁺ | 62 | 66 |
| Ring: [C₆H₄]⁺ | 76 | 80 |
| [C₆H₄-CO]⁺ | 104 | 108 |
| [C₆H₄-COO-CH₂-CH₂]⁺ | 148 | 156 |
| [C₆H₄-COO-CO]⁺ | 149 | 153 |
| Molecular ion [M]⁺ | 193 | 201 |

In the case of 50% of marked positions in both precursor monomers, the protium and deuterium signals appear in the chromatogram with same intensity. Since the isotopic differences do not affect significantly the interaction of compounds with the chromatographic column, the separation was not possible (even for longer and soft methods) and the retention time was almost the same.

To solve this, the GC/MS systems allows the selective plot of the preferred masses. Protium and deuterium signals (table 3) could be plotted separately and compare the number of accumulated counts of every species.

The ratio of the intensity of the accumulated counts of the masses detected must be proportional to the ratio of the marked:non-marked positions.

### Example 5: identification of functional additives

Three additives of common polymeric use are presented as examples of identification by GC/MS. The methodology of study and identification will be the same as for PET:

### Dioctyl phthalate (DOP)

DOP is used as plasticizer.

### Gas chromatography and mass spectrometry (GC/MS)

The +1(M/z) caused by every deuterium introduced instead of a protium has an accumulative effect, and in the Dioctyl-Phthalate-d₃₈ (DOP) case, the 38 marked positions generates a +38 (M/z) for the molecular ion, and at least +4(M/z) in the most of identification fragments:

**Table 4. Summary of main differences in the mass (M/z) of fragments that can be detected by marking the DOP.**

| **Fragment** | **Protium (cm⁻¹)** | **Deuterium (cm⁻¹)** |
|---|---|---|
| [(CH₂)₂-CH₃]⁺ | 43 | 50 |
| [(CH₂)₃-CH₃]⁺ | 57 | 66 |
| [(CH₂)₄-CH₃]⁺ | 71 | 82 |
| Ring: [C₆H₄]⁺ | 76 | 80 |
| [C₆H₄-COO-CO]⁺ | 149 | 153 |
| [C₆H₄-COO(CH₂)₇CH₃-COO]⁺ | 279 | 300 |
| Molecular ion [M]⁺ | 390 | 428 |

### Decamethyltetrasiloxane

Decamethyltetrasiloxane is used as adsitive in adhesives and lubricants.

### Gas chromatography and mass spectrometry (GC/MS)

The +1(M/z) caused by every deuterium introduced instead of a protium has an accumulative effect, and in the decamethyltetrasiloxane-d₃₀ case, the 30 marked positions generates a +30 (M/z) for the molecular ion, and at least +9(M/z) in the most of identification fragments:

**Table 5. Summary of main differences in the mass (M/z) of fragments that can be detected by marking the decamethyltetrasiloxane.**

| **Fragment** | **Protium (cm⁻¹)** | **Deuterium (cm⁻¹)** |
|---|---|---|
| [(CH₃)₃Si]⁺ | 73 | 82 |
| [(CH₃)₃Si-(CH₃)₂SiO]⁺ | 147 | 162 |
| [((CH₃)₂SiO)₂-(CH3)SiO₂]⁺ | 207 | 222 |
| [(CH₃)₃Si-((CH₃)₂SiO)₃]⁺ | 295 | 322 |
| [M]⁺ | 310 | 340 |

### Benzotriazole

Benzotriazole is used as UV photostabilizer.

### Gas chromatography and mass spectrometry (GC/MS)

The +1 (M/z) caused by every deuterium introduced instead of a protium has an acumulative effect, and in the Benzotriazole-d₄ case, the 4 marked positions generates a +4 (M/z) for the molecular ion, and at least +3(M/z) in the most of identification fragments:

**Table 6.- Summary of main differences in the mass (M/z) of fragments that can be detected by marking the decamethyltetrasiloxane.**

| **Fragment** | **Protium (cm⁻¹)** | **Deuterium (cm⁻¹)** |
|---|---|---|
| [(CH)₃]⁺ | 39 | 42 |
| [(CH)₄]⁺ | 52 | 56 |
| [(CH)₄-C]⁺ | 64 | 68 |
| [(CH)₄-C₂-NH]⁺ | 91 | 95 |
| [M]⁺ | 119 | 124 |

## Claims

1. Use of a material comprising a synthetic functional polymer and optionally at least one functional additive in the detection of material contamination or degradation or wear, wherein said material is marked with at least one isotope, wherein the isotope or isotopes are present in a functional component of the material, wherein the term "functional" has the meaning as defined in the description, and wherein the at least one isotope is selected from ²H, ¹³C, ¹⁵N, ¹⁷O, ¹⁸O, ²⁹Si, ³⁰Si, ³³S, ³⁴S, ³⁶S, ³⁷Cl, wherein said material is a space material or an industrial material, preferably wherein the industrial material is a material to be used in a clean room.

2. The use according to the preceding claim, wherein said material comprises more than one component and wherein the same isotope is used for marking different components.

3. The use according to claim 1, wherein said material comprises more than one component and wherein a different isotope is used for marking different components.

4. The use according to any one of the preceding claims, wherein the isotope is introduced in a specific position in a monomer of the synthetic polymer.

5. The use according to any one of the preceding claims, wherein at least 0.3 % of the atoms of the chemical element of the isotope are marked, in respect of the total number of atoms of that chemical element in the marked component of the material.

6. The use according to any one of the preceding claims, wherein the isotopic mark is detected by FTIR, Raman, GC/MS, RMN-H, RMN-C, UV-visible spectroscopy.

7. The use according to any one of the preceding claims, wherein the synthetic polymer is an addition polymer or a condensation polymer.

8. The use according to any one of the preceding claims, wherein the synthetic polymer is a polyolefin, a polyester, a polyurethane, a polyimide, a polyacrylate, a polysiloxane, a polyepoxide, a fluorinated polymer or a combination thereof.

9. The use according to any one of the preceding claims, wherein the synthetic polymer is polyethylene (PE), polyethylene terephthalate (PET), polyamide (PA), ethylene tetrafluoroethylene (ETFE), polytetrafluoroethylene (PTFE), perfluoroalkoxy alkane (PFA), polyetheretherketone (PEEK), polyethersulphone (PES), polysulfone, polyetherimide (PEI) or a copolymer o terpolymer thereof.

10. The use according to any one of the preceding claims, wherein the material is used as a calibrator for a Raman spectrometer.

11. The use according to any of the preceding claims, wherein the material is comprised in a composite material.

12. The use according to the preceding claim, wherein the composite material comprises at least one of carbon fibre, polyethylene, polypropylene, nylon or kevlar.

## Patentansprüche

1. Verwendung eines Materials, umfassend ein synthetisches funktionelles Polymer und gegebenenfalls mindestens einen funktionellen Zusatzstoff, zur Erkennung von Materialverunreinigung oder -abbau oder verschleiß, wobei das Material mit mindestens einem Isotop markiert ist, wobei das Isotop oder die Isotope in einer funktionellen Komponente des Materials vorhanden sind, wobei der Begriff "funktionell" die in der Beschreibung definierte Bedeutung hat und wobei das mindestens eine Isotop aus ²H, ¹³C, ¹⁵N, ¹⁷O, ¹⁸O, ²⁹Si, ³⁰Si, ³³S, ³⁴S, ³⁶S, ³⁷Cl ausgewählt ist, wobei das Material ein Weltraummaterial oder ein Industriematerial ist, wobei das Industriematerial vorzugsweise ein Material ist, das in einem Reinraum zu verwenden ist.

2. Verwendung nach dem vorhergehenden Anspruch, wobei das Material mehr als eine Komponente umfasst und wobei dasselbe Isotop zum Markieren unterschiedlicher Komponenten verwendet wird.

3. Verwendung nach Anspruch 1, wobei das Material mehr als eine Komponente umfasst und wobei ein unterschiedliches Isotop zum Markieren unterschiedlicher Komponenten verwendet wird.

4. Verwendung nach einem der vorhergehenden Ansprüche, wobei das Isotop in einer spezifischen Position in ein Monomer des synthetischen Polymers eingeführt wird.

5. Verwendung nach einem der vorhergehenden Ansprüche, wobei mindestens 0,3 % der Atome des chemischen Elements des Isotops markiert sind, bezogen auf die Gesamtzahl der Atome dieses chemischen Elements in der markierten Komponente des Materials.

6. Verwendung nach einem der vorhergehenden Ansprüche, wobei die Isotopenmarkierung durch FTIR, Raman, GC/MS, RMN-H, RMN-C, UV-sichtbare Spektroskopie erkannt wird.

7. Verwendung nach einem der vorhergehenden Ansprüche, wobei das synthetische Polymer ein Additionspolymer oder ein Kondensationspolymer ist.

8. Verwendung nach einem der vorhergehenden Ansprüche, wobei das synthetische Polymer ein Polyolefin, ein Polyester, ein Polyurethan, ein Polyimid, ein Polyacrylat, ein Polysiloxan, ein Polyepoxid, ein fluoriertes Polymer oder eine Kombination davon ist.

9. Verwendung nach einem der vorhergehenden Ansprüche, wobei das synthetische Polymer Polyethylen (PE), Polyethylenterephthalat (PET), Polyamid (PA), Ethylentetrafluorethylen (ETFE), Polytetrafluorethylen (PTFE), Perfluoralkoxialkan (PFA), Polyetheretherketon (PEEK), Polyethersulfon (PES), Polysulfon, Polyetherimid (PEI) oder ein Copolymer oder Terpolymer davon ist.

10. Verwendung nach einem der vorhergehenden Ansprüche, wobei das Material als ein Kalibrator für ein Raman-Spektrometer verwendet wird.

11. Verwendung nach einem der vorhergehenden Ansprüche, wobei das Material in einem Verbundmaterial umfasst ist.

12. Verwendung nach dem vorhergehenden Anspruch, wobei das Verbundmaterial mindestens eines von Kohlenstofffaser, Polyethylen, Polypropylen, Nylon oder Kevlar umfasst.

## Revendications

1. Utilisation d'un matériau comprenant un polymère synthétique fonctionnel et éventuellement au moins un additif fonctionnel dans la détection d'une contamination ou d'une dégradation ou d'une usure de matériau, dans laquelle ledit matériau est marqué avec au moins un isotope, dans laquelle l'isotope ou les isotopes sont présents dans un composant fonctionnel du matériau, dans laquelle le terme "fonctionnel" a la signification telle que définie dans la description, et dans laquelle l'au moins un isotope est choisi parmi ²H, ¹³C, ¹⁵N, ¹⁷O, ¹⁸O, ²⁹Si, ³⁰Si, ³³S, ³⁴S, ³⁶S, ³⁷Cl, dans laquelle ledit matériau est un matériau spatial ou un matériau industriel, de préférence dans laquelle le matériau industriel est un matériau pour être utilisé dans une salle blanche.

2. Utilisation selon la revendication précédente, dans laquelle ledit matériau comprend plus d'un composant et dans laquelle le même isotope est utilisé pour marquer différents composants.

3. Utilisation selon la revendication 1, dans laquelle ledit matériau comprend plus d'un composant et dans laquelle un isotope différent est utilisé pour marquer différents composants.

4. Utilisation selon l'une quelconque des revendications précédentes, dans laquelle l'isotope est introduit dans une position spécifique dans un monomère du polymère synthétique.

5. Utilisation selon l'une quelconque des revendications précédentes, dans laquelle au moins 0,3% des atomes de l'élément chimique de l'isotope sont marqués, par rapport au nombre total d'atomes de cet élément chimique dans le composant marqué du matériau.

6. Utilisation selon l'une quelconque des revendications précédentes, dans laquelle le marqueur isotopique est détecté par spectroscopie FTIR, Raman, GC/MS, RMN-H, RMN-C, UV-visible.

7. Utilisation selon l'une quelconque des revendications précédentes, dans laquelle le polymère synthétique est un polymère d'addition ou un polymère de condensation.

8. Utilisation selon l'une quelconque des revendications précédentes, dans laquelle le polymère synthétique est une polyoléfine, un polyester, un polyuréthane, une polyimide, un polyacrylate, un polysiloxane, un polyépoxide, un polymère fluoré ou une combinaison de ceux-ci.

9. Utilisation selon l'une quelconque des revendications précédentes, dans laquelle le polymère synthétique est du polyéthylène (PE), du poly(téréphtalate d'éthylène) (PET), de la polyamide (PA), du éthylène-tetrafluoroéthylène (ETFE), du polytetrafluoroéthylène (PTFE), du perfluoroalkoxy-alcane (PFA), de la polyétheréthercétone (PEEK), de la polyéthersulfone (PES), de la polysulfone, de la polyétherimide (PEI) ou un copolymère o terpolymère de ceux-ci.

10. Utilisation selon l'une quelconque des revendications précédentes, dans laquelle le matériau est utilisé comme étalonneur pour un spectromètre de Raman.

11. Utilisation selon l'une quelconque des revendications précédentes, dans laquelle le matériau est compris dans un matériau composite.

12. Utilisation selon la revendication précédente, dans laquelle le matériau composite comprend au moins l'un de fibre de carbone, polyéthylène, polypropylène, nylon ou kevlar.
